Europäisches Patentamt

European Patent Office

Office européen des brevets

(11) Publication number: **0 342 030**
**A2**

(12) **EUROPEAN PATENT APPLICATION**

(21) Application number: **89304773.8**

(22) Date of filing: **11.05.89**

(51) Int. Cl.⁴: **A 61 K 31/445**

(30) Priority: **12.05.88 GB 8811311**

(43) Date of publication of application:
**15.11.89 Bulletin 89/46**

(84) Designated Contracting States:
**AT BE CH DE FR GB IT LI NL SE**

(71) Applicant: **GLAXO GROUP LIMITED**
**Clarges House 6-12 Clarges Street**
**London W1Y 8DH (GB)**

(72) Inventor: **Woodings, David Francis**
**Cranmer Lodge Maybush Lane**
**Felixstowe Suffolk IP11 7QN (GB)**

(74) Representative: **Holmes, Michael John et al**
**Frank B. Dehn & Co. Imperial House 15-19 Kingsway**
**London WC2B 6UZ (GB)**

(54) Use of a thromboxane receptor antagonist to complement arterial surgery.

㊼ The use is described of [1R-[1α(Z),2β,3β,5α]]--(+)-7-[5-[[(1,1'-biphenyl)-4-yl]methoxy]-3-hydroxy-2-(1-piperidinyl)cyclopentyl]-4-heptenoic acid or a physiologically acceptable salt, solvate or cyclodextrin complex thereof in the manufacture of medicaments to compliment the surgical removal of an obstruction in an intact artery.

EP 0 342 030 A2

Bundesdruckerei Berlin

**Description**

## USE OF A THROMBOXANE RECEPTOR ANTAGONIST TO COMPLIMENT ARTERIAL SURGERY

This invention relates to a new medical use for [1R-[1α(Z),2β,3β,5α]]-(+)-7-[5-[[(1,1'-biphenyl)-4-yl]methoxy]-3-hydroxy-2-(1-piperidinyl)cyclopentyl]-4-heptenoic acid (hereinafter referred to as Compound A) or a physiologically acceptable salt, solvate or cyclodextrin complex thereof. In particular, it relates to the use of Compound A or a physiologically acceptable salt, solvate or cyclodextrin complex thereof to compliment the surgical removal of an obstruction in an intact artery.

As alternatives to bypass grafting, surgical techniques have been devised to remove obstructions in an artery, however leaving the artery intact. Such techniques include percutaneous transluminal angioplasty (using, for example, balloons, lasers or rotational devices) and endarterectomy (including gas and laser endarterectomy). However, despite the initial high success rates of these alternative techniques (which are hereinafter referred to under the general heading "angioplasty"), in many cases the arterial lumen narrows again within a short time. Indeed, in patients who have received percutaneous transluminal coronary angioplasty the arterial lumen narrows again by six months in 20 to 40% of cases. The process of restenosis and reocclusion is thought to begin either with an initial thrombotic occlusion immediately after the procedure or by secondary occlusion some months later due to thickening of the intima.

Our UK Patent Specification 2097397 describes inter alia Compound A which is a potent thromboxane receptor blocker. We have stated therein that the compounds of the invention, including Compound A, inhibit thromboxane $A_2$ and endoperoxide mediated aggregation of blood platelets and contraction of vascular smooth muscle and are thus of particular interest as anti-thrombotic agents.

We have now found that Compound A or a physiologically acceptable salt, solvate or cyclodextrin complex thereof can reduce reocclusion, restenosis and thromboxane-induced vasospasm of an artery (e.g. a coronary, carotid or femoral artery) following the surgical removal of an obstruction in said intact artery, e.g. by angioplasty.

Thus, according to one aspect of the present invention, we provide Compound A or a physiologically acceptable salt, solvate or cyclodextrin complex thereof for use in the manufacture of a medicament to reduce reocclusion, restenosis and thromboxane-induced vasospasm of an artery of a human or animal subject following the surgical removal of an obstruction in said intact artery.

In another aspect of the present invention, we provide the use of Compound A or a physiologically acceptable salt, solvate or cyclodextrin complex thereof to reduce reocclusion, restenosis and thromboxane-induced vasospasm of an artery of a human or animal subject following the surgical removal of an obstruction in said intact artery.

It is preferable to employ Compound A or a physiologically acceptable salt, solvate or cyclodextrin complex thereof in the form of a pharmaceutical formulation. The present invention therefore also provide a pharmaceutical composition for use in human or veterinary medicine to reduce reocclusion, restenosis and thromboxane-induced vasospasm of an artery of a human or animal subject following surgical removal of an obstruction in said intact artery comprising Compound A or a physiologically acceptable salt, solvate or cyclodextrin complex thereof together, where desirable, with one or more pharmaceutical carriers or excipients.

The term "obstruction" means herein the complete blockage or a narrowing of the arterial lumen.

In a further aspect of the invention, we provide a method of treatment of a human or animal subject suffering from a disease associated with an obstruction in an intact artery which method comprises the surgical removal of said obstruction (e.g. by angioplasty) and administration to said subject of Compound A or a physiologically acceptable salt, solvate or cyclodextrin complex thereof in an amount sufficient to reduce reocclusion, restenosis and thromboxane-induced vasospasm of said artery.

It will be appreciated that Compound A or a physiologically acceptable salt, solvate or cyclodextrin complex may be administered before, during or after surgery. Conveniently, the patient is given a loading dose of Compound A or a physiologically acceptable salt, solvate or cyclodextrin complex, and after surgery treatment with Compound A or a physiologically acceptable salt, solvate or cyclodextrin complex is continued to maximise inhibition of post-surgical restenosis and reocclusion. Thus, for example, oral maintenance therapy may be continued, e.g. for six months.

In another aspect of the present invention, we provide a method for maintaining patency of an artery of a human or animal subject after the surgical removal of an obstruction in the intact artery (e.g. after angioplasty) which comprises administering to said subject Compound A or a physiologically acceptable salt, solvate or cyclodextrin complex thereof in an amount sufficient to maintain patency of the said artery.

Stents are sometimes used, after surgical removal of an obstruction in an intact artery, to prevent restenosis at the site of the original stenosis. However, the use of these devices (which are usually fashioned from stainless steel or plastic) has been plagued by a high reocclusion rate. It is now envisaged that this reocclusion rate will be significantly reduced by appropriate administration of Compound A or a physiologically acceptable salt, solvate or cyclodextrin complex thereof.

In a further aspect of the present invention therefore, we provide Compound A or a physiologically acceptable salt, solvate or cyclodextrin complex thereof for use in the manufacture of a medicament to reduce reocclusion of an artery of a human or animal subject following the surgical removal of an obstruction in said intact artery and insertion of a stent at the site of stenosis.

Compound A and physiologically acceptable salts, solvates and cyclodextrin complexes thereof may be of particular benefit when used according to the present invention to treat patients suffering from ischaemic heart disease.

Thus, in a particular embodiment of the present invention, we provide a method of treatment of a human or animal subject suffering from ischaemic heart disease which method comprises the surgical removal of an obstruction in an intact coronary artery (e.g. by angioplasty) and administration of Compound A or a physiologically acceptable salt, solvate or cyclodextrin complex thereof in an amount sufficient to reduce reocclusion, restenosis and thromboxane-induced vasospasm of said artery.

In a preferred aspect of the present invention, Compound A or a physiologically acceptable salt, solvate or cyclodextrin complex thereof is used to compliment coronary angioplasty, especially percutaneous transluminal coronary angioplasty.

Suitable salts of Compound A include acid addition salts derived from inorganic and organic acids such as hydrochlorides, hydrobromides, sulphates, phosphates, maleates, tartrates, citrates, benzoates, 2-chlorobenzoates, p-toluenesulphonates, methanesulphonates, salicylates, fumarates, lactates, hydroxynaphthalenecarboxylates (e.g. 1-hydroxy- or 3-hydroxy-2-naphthalenecarboxylates) or furoates, or salts with suitable bases such as alkali metal (e.g. sodium and potassium), alkaline earth metal (e.g. calcium or magnesium), ammonium end substituted ammonium (e.g. dimethylammonium, triethylammonium, 2-hydroxyethyldimethylammonium, piperazinium, N,N-dimethylpiperazinium, piperidinium, ethylenediaminium and choline) salts. A preferred salt of Compound A is the hydrochloride salt. Compound A and physiologically acceptable salts and solvates thereof and pharmaceutical formulations containing them are described in GB-B-2097397 and GB-B-2127406.

When Compound A is used according to the present invention in the form of a cyclodextrin complex, the complex conveniently contains a molar ratio of Compound A with cyclodextrin within the range 1:1 to 1:3.

The term "cyclodextrin" means herein an unsubstituted or substituted α-, β- or γ-cyclodextrin (or a hydrate thereof) or a mixture of two or three of them. Examples of suitable substituted cyclodextrins include sulphur-containing cyclodextrins, nitrogen-containing cyclodextrins, alkylated (e.g. methylated) cyclodextrins such as mono-, di- or trimethylated derivatives of a cyclodextrin (e.g. of β-cyclodextrin) and hydroxyalkyl (e.g. hydroxypropyl) cyclodextrins such as hydroxypropyl β-cyclodextrin and acylated derivatives thereof. Hydroxyalkyl (e.g. hydroxypropyl) cyclodextrins such as hydroxypropyl β-cyclodextrin have been found to be particularly suitable.

A particularly preferred cyclodextrin complex of Compound A is the β-cyclodextrin complex in which the molar ratio of Compound A with β-cyclodextrin is about 1:1.

Cyclodextrin complexes of Compound A and pharmaceutical formulations containing them are described in our co-pending British Patent Application No. 8829793. The cyclodextrin complexes may be prepared by mixing Compound A or the hydrochloride salt thereof with the cyclodextrin in a suitable solvent such as water or an organic solvent which is miscible with water (e.g. an alcohol such as methanol). The reaction may take place at any temperature in the range from 0° to 80°C. However, the mixture is preferably kept at around room temperature and the desired complex obtained by concentrating the mixture under reduced pressure or by allowing the mixture to cool. The mixing ratio of organic solvent with water may be suitably varied according to the solubilities of the starting materials and products. Preferably 1 to 4 moles of cyclodextrin are used for each mole of Compound A or its hydrochloride salt.

Pharmaceutical formulations containing a cyclodextrin complex of Compound A may be prepared in conventional manner by mixing the complex with one or more pharmaceutical carriers or excipients, for example, according to the general methods described in GB-B-2097397 and GB-B-2127406.

When Compound A or a physiologically acceptable salt, solvate or cyclodextrin complex is to be administered as an aqueous formulation for, in particular, parenteral (e.g. intravenous) use, the composition may be prepared according to the general methods described in GB-B-2097397 and GB-B-2127406. Alternatively, the aqueous compositions may be prepared by mixing Compound A or, more preferably, the hydrochloride salt thereof with cyclodextrin together with one or more pharmaceutical carriers or excipients, for example as described in the Examples hereinafter. Preferably, Compound A or its hydrochloride salt are dissolved in water and the remaining constituents are added thereto.

The molar ratio of Compound A or its hydrochloride salt with cyclodextrin in the aqueous composition is conveniently within the range 1:1 to 1:3.

Preferably, the aqueous formulation comprises the hydrochloride salt of Compound A and β-cyclodextrin (or a hydrate thereof) at about physiological pH wherein the formulation contains at least about 1.2 moles of β-cyclodextrin (e.g. 1.2 to 2 moles) for every one mole of the hydrochloride salt of Compound A. Preferably the molar ratio will be about 1:1.4.

The precise dose of Compound A or a physiologically acceptable salt, solvate or cyclodextrin complex thereof to be administered will, of course, depend on a number of factors including, for example, the age and weight of the patient and the route of administration. An effective oral dose, however, in the case of Compound A or a physiologically acceptable salt, solvate or cyclodextrin complex thereof is likely to be in the range from 0.05 to 20 mg/kg body weight of patient per day, preferably in the range from 0.05 to 5 mg/kg per day. If administered before or after surgery, the pre-surgical dose of Compound A or a physiologically acceptable salt, solvate or cyclodextrin complex thereof is likely to be a single loading dose in the range of 0.05 to 5mg/kg.

The concentration of Compound A or the hydrochloride salt thereof in the aforementioned aqueous formulations suitable for parenteral administration, in particular for administration by injection (eg intravenously), is conveniently within the range 0.1-10mg/ml, e.g. 0.1-5mg/ml, expressed as the free base. Preferably, the concentration is 1mg/ml expressed as the free base when the aqueous formulation is administered by intravenous injection. If desired, a higher concentration may be used and the solution may be diluted prior to use with, for example, an isotonic saline solution or dextrose or mannitol solution. Conveniently, solutions suitable for injection are presented in an appropriate dose volume (eg 1 - 100 ml). Dilutions suitable for continuous infusion may have a concentration of Compound A or its hydrochloride salt of 0.01 - 0.2 mg/ml expressed as the free base. The solution for continuous infusion may be presented in this form, for example in packs of 50-100 ml, or may be presented in more concentrated forms for subsequent dilution before use with, for example, an isotonic saline solution or dextrose or mannitol solution. Alternatively, small volumes of a more concentrated solution (eg 0.1 - 5 mg/ml) may be utilised for continuous infusion conveniently administered at a rate of 0.5 to 9.9 ml/h.

The aforementioned aqueous formulations may also be adapted for oral administration (e.g. as a capsule, syrup or solution). The preparation of suitable formulations for oral use will be within the knowledge of persons skilled in the art and may generally follow the procedures described in GB-B-2097397, GB-B-2127406 and in the Examples hereinafter.

The following examples are provided in illustration of the present invention and should not be construed in any way as constituting a limitation thereof.

Pharmaceutical examples of parenteral injections/infusions comprising the hydrochloride salt of Compound A

(i) Hydrochloride Salt of Compound A equivalent to 50 mg base

| β-Cyclodextrin hydrate | 143mg | 166mg | 238mg |
|---|---|---|---|
| Sodium hydroxide solution | to pH7 | to pH7 | to pH7 |
| Water suitable for injection | to 50ml | to 50ml | to 50ml |

The hydrochloride salt of Compound A was dissolved in 35ml water suitable for injection and the β-cyclodextrin was added. This solution was titrated to pH7 with 0.02M sodium hydroxide solution and then adjusted to volume with water suitable for injection.

The solution may then be sterilised by filtration and filled into vials or ampoules.

(ii) Hydrochloride salt of Compound A equivalent to 50mg base

| β-Cyclodextrin hydrate | 166mg |
|---|---|
| Sodium chloride | 450mg |
| pH7.0 phosphate buffer | 2.5ml |
| Sodium hydroxide solution | to pH7 |
| Water suitable for injection | to 50ml |

The hydrochloride salt of Compound A was dissolved in approximately 25ml water suitable for injection. The β-cyclodextrin was dissolved therein and the resulting solution was titrated to pH6 with 0.02M sodium hydroxide solution and the phosphate buffer added. The sodium chloride was added to the solution and the pH adjusted to pH7 with sodium hydroxide. The solution was made up to volume with water suitable for injection. A sample of this solution was filled into a glass vial which was sealed with a rubber plug and metal overseal. This was then autoclaved.

(iii) Hydrochloride salt of Compound A equivalent to 50 mg base

4

| Hydroxypropyl-β-cyclo-dextrin | 170mg |
|---|---|
| Mannitol | 2.5g |
| pH 6.0 phosphate buffer | 5.0ml |
| Sodium hydroxide solution | to pH 6 |
| Water suitable for injection | to 50ml |

The hydrochloride salt of Compound A was dissolved in approximately 25ml water suitable for injection and the hydroxypropyl-β-cyclodextrin was added. The mannitol was then added and the solution titrated to pH 6 with 0.02M sodium hydroxide solution. The phosphate buffer solution was added and the solution adjusted to volume with water suitable for injection. The solution was then filtered and filled into glass vials which were sealed with rubber plugs and metal overseals. These were then autoclaved.

(iv) Hydrochloride salt of Compound A equivalent to 50mg base

| β-Cyclodextrin hydrate | 166mg |
|---|---|
| Mannitol | 2.5g |
| Sodium acid phosphate | 46mg |
| Disodium phosphate, anhydrous | 5mg |
| Sodium hydroxide solution | to pH 6 |
| Water suitable for injection | to 50ml |

The hydrochloride salt of Compound A was dissolved in approximately 25ml water suitable for injection. The β-cyclodextrin and mannitol were dissolved therein and the solution titrated to pH 6 with 0.02M sodium hydroxide solution. The sodium acid phosphate and anhydrous disodium phosphate were dissolved in water suitable for injection. This solution was added to the bulk solution which was made up to volume with water suitable for injection. The solution was filtered and filled into glass ampoules which were sealed and then autoclaved.

(v)

|  | α | γ | Cyclodextrin Mixture β + γ | |
|---|---|---|---|---|
| Hydrochloride salt of Compound A equivalent to 50mg base |  |  |  |  |
| Cyclodextrin | 143mg | 190mg | 119mg | 136mg |
| Mannitol | 2.5g | 2.5g |  | 2.5g |
| pH 6.0 Phosphate buffer | 5.0ml | 5.0ml |  | 5.0ml |
| Sodium hydroxide solution | to pH 6 | to pH 6 |  | to pH 6 |
| Water suitable for injection | to 50ml | to 50 ml |  | to 50ml |

The hydrochloride salt of Compound A was dissolved in approximately 25ml water suitable for injection and the cyclodextrin(s) was (were) added. The mannitol was then added and the solution titrated to pH 6 with 0.02M sodium hydroxide solution. The phosphate buffer solution was added and the solution was adjusted to volume with water suitable for injection. The solution was then filtered and filled into glass vials which were sealed with rubber plugs and metal overseals.

Pharmaceutical example of an oral syrup comprising the hydrochloride salt of Compound A

5

Hydrochloride salt of Compound A equivalent to 2.5mg base

| | |
|---|---|
| β-cyclodextrin hydrate | 9mg |
| Citric acid | to pH 4.5 |
| Methyl hydroxybenzoate sodium | 5mg |
| Propyl hydroxybenzoate sodium | 2mg |
| Liquid orange flavour | qs |
| Sucrose | 3.25g |
| Purified water | to 5.0ml |

Dissolve the sucrose in a minimum quantity of water. Add the hydrochloride salt of Compound A and then the β-cyclodextrin with stirring; adjust the pH to 4.5 with citric acid. With continued stirring add a solution of the hydroxybenzoates and lastly the flavour. Adjust almost to volume with water and stir. Check the pH and adjust to 4.5 with citric acid if necessary. Make up to volume with water.

.

## Claims

1. Use of [IR-[1α(Z),2β,3β,5α]]-(+)-[5-[[(1,1'-biphenyl)-4-yl] methoxy]-3-hydroxy-2-(1-piperidinyl)-cyclopentyl]-4-heptenoic acid (Compound A) or a physiologically acceptable salt, solvate or cyclodextrin complex thereof in the manufacture of medicaments for reduction of thromboxane-induced vasospasm and/or maintenance of patency of an intact artery of a human or animal subject following the surgical removal of an obstruction in said artery, including reduction of reocclusion and/or restenosis of said artery.

2. Use as claimed in claim 1 wherein Compound A or a physiologically acceptable salt, solvate or cyclodextrin complex thereof is used in the manufacture of medicaments for reduction of reocclusion of an artery of a human or animal subject following the surgical removal of an obstruction in said intact artery and insertion of a stent at the site of stenosis.

3. Use as claimed in Claim 1 or Claim 2 for the treatment of ischaemic heart disease.

4. Use as claimed in any preceding claim in which Compound A is in the form of its hydrochloride salt.

5. Use as claimed in any preceding claim in which the medicament claimed is in a form suitable for oral or parenteral administration.

6. Use as claimed in claim 5 wherein said medicament comprises compound A or a physiologically acceptable salt, solvate or cyclodextrin complex thereof in an amount sufficient to administer 0.05 to 20 mg/kg body weight per day to said human or animal subject.

7. Use as claimed in any preceding claim in which the surgical removal of an obstruction in an intact artery is effected by coronary angioplasty.

8. Use as claimed in any preceding claim in which the surgical removal of an obstruction in an intact artery is effected by percutaneous transluminal coronary angioplasty.